# EUROPEAN PATENT APPLICATION

(11) **EP 2 193 768 A1**
(43) Date of publication of application: **09.06.2010**
(21) Application number: 09173164.6
(22) Date of filing: 15.10.2009
(51) Int. Cl.: A61F 9/06

(54) **Welding mask that can control LCD shielding goggle without taking off**

(30) Priority: 03.12.2008 KR 20080121577
(71) Applicant: Otos Tech Co., Ltd., Hwaseoung-city Gyeonggi-do (KR)
(72) Inventor: Huh, Moon Young, 153-801, Seoul (KR)
(74) Representative: Ihle, Kornelia

(57) **Abstract**

Disclosed is a welding mask including a welding face to cover the user's face, the welding face having a transparent window, a wearing band to be worn on the user's hair, a liquid crystal display (LCD) shielding goggle (310) including an LCD panel mounted at the rear of the transparent window of the welding face to protect the user's eyes from strong injurious light rays generated during welding, and a manipulation unit (400) disposed spaced apart from the lower end of the LCD shielding goggle.

## Description

### BACKGROUND OF THE INVENTION

### Field of the Invention

The present invention relates to a welding mask, and, more particularly, to a welding mask that enables a user to easily and conveniently perform manipulation thereof, thereby protecting the user's eyes and face.

### Description of the Related Art

Generally, strong injurious light rays are generated during various kinds of welding. In addition, parent metal may be spattered to a user's face due to instantaneously high heat and resistance.

For this reason, it is a matter of common knowledge that a welding mask is used to protect the eyes and face of a worker from the above-described risks during welding.

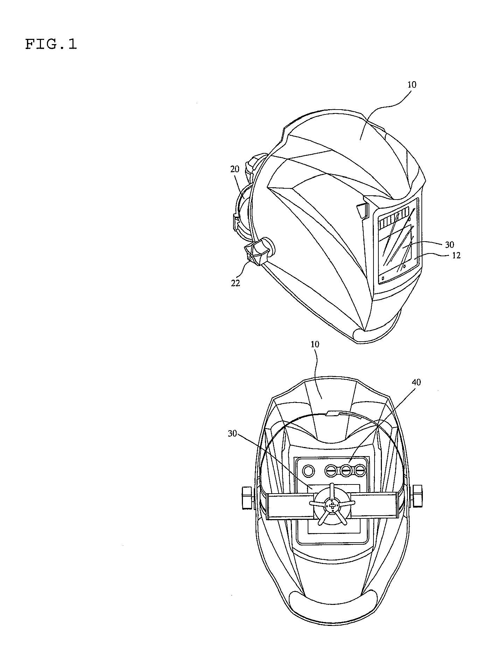

FIG. 1 is a perspective view showing a conventional welding mask.

As shown in FIG. 1, the conventional welding mask includes a welding face 10 configured to cover the entirety of a user's face, the welding face 10 having a predetermined transparent window 12 formed at the front thereof, a wearing band 20 configured to be worn on the user's hair, the wearing band 20 being coupled to the welding face 10 via pivot shafts 22 such that the welding face 10 is rotated upward/downward by a predetermined angle, and a liquid crystal display (LCD) shielding goggle 30 mounted at the rear of the transparent window 12 of the welding face 10 to protect the user's eyes from strong injurious light rays generated during welding.

Although not shown in the drawing, on the other hand, the LCD shielding goggle 30 includes an LCD panel to allow the user to see through the front while shielding injurious light rays, a manipulation unit to detect injurious light rays generated during welding and to operate the LCD panel, and a power supply unit to power to the LCD panel. The LCD panel, the manipulation unit, and the power supply unit are integrally mounted in a case.

Therefore, the LCD shielding goggle 30 is in tight contact with the rear of the transparent window 12 of the welding face 10, as previously described, and, at the same time, various manipulators 40 of the manipulation unit are disposed at the rear of the transparent window 12 of the welding face 10 such that the user adjusts sensitivity and shielding timing.

In the conventional welding mask as described above, however, it is necessary for the user to take off the welding face 10 to manipulate the LCD shielding goggle 30 whenever it is necessary to adjust sensitivity and shielding timing, since the LCD shielding goggle 30 and the various manipulators 40 are disposed at the rear of the transparent window 12 of the welding face 10, which is very inconvenient.

### SUMMARY OF THE INVENTION

Therefore, the present invention has been made in view of the above problems, and it is an object of the present invention to provide a welding mask that can control a liquid crystal display (LCD) shielding goggle without taking off, which enables a user to adjust sensitivity and shielding timing without taking off a welding face, thereby achieving easy and convenient user manipulation.

In accordance with the present invention, the above and other objects can be accomplished by the provision of a welding mask that can control an LCD shielding goggle without taking off, including a welding face configured to cover the entirety of a user's face, the welding face having a predetermined transparent window formed at the front thereof, a wearing band configured to be worn on the user's hair, the wearing band being coupled to the welding face via pivot shafts such that the welding face is rotated upward/downward by a predetermined angle, an LCD shielding goggle including an LCD panel mounted at the rear of the transparent window of the welding face to protect the user's eyes from strong injurious light rays generated during welding, and a manipulation unit disposed spaced apart by a predetermined distance from the lower end of the LCD shielding goggle, the manipulation unit serving to detect injurious light rays generated during welding and operate the LCD panel accordingly.

The LCD panel and the manipulation unit may be connected to each other in a wire communication fashion.

The LCD panel and the manipulation unit may be connected to each other in a radio communication fashion.

The manipulation unit may be configured in the form of a touch panel using an LCD or light emitting diodes (LED).

### BRIEF DESCRIPTION OF THE DRAWINGS

The above and other objects, features and other advantages of the present invention will be more clearly understood from the following detailed description taken in conjunction with the accompanying drawings, in which:
FIG. 1 is a perspective view showing a conventional welding mask;
FIG. 2 is a front view showing a welding mask according to an embodiment of the present invention;
FIG. 3 is a front view showing a welding mask according to another embodiment of the present invention;
FIG. 4 is a front view showing a welding mask according to a further embodiment of the present invention;
FIG. 5 is a view showing the use state of the welding mask according to the present invention; and
FIG. 6 is a block diagram showing a control circuit of the welding mask according to the present invention.

### DETAILED DESCRIPTION OF THE INVENTION

Now, exemplary embodiments of the present invention will be described in detail with reference to the accompanying drawings.

It should be understood that terms and words used in the specification and claims are not to be construed as having common and dictionary meanings, but correspond to the technical spirit of the present invention in view of the principle that the inventor can define proper terms in order to describe his/her invention as best possible. While embodiments described in the specification and the construction thereof shown in the accompanying drawings are simply the most exemplary embodiments of the invention, they do not completely encompass the technical spirit of the invention. Therefore, it should be understood that various modifications and equivalents capable of replacing the disclosed embodiment could be made on the filing date of the application.

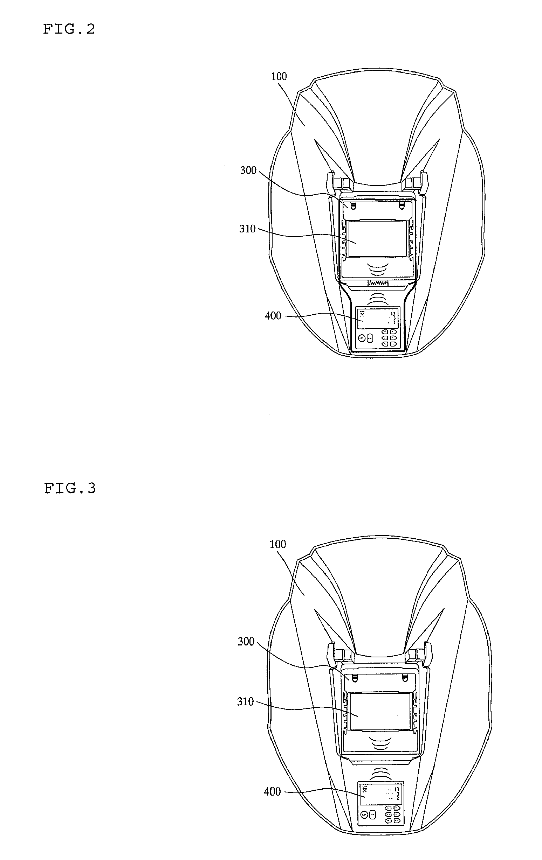

FIG. 2 is a front view showing a welding mask that can control a liquid crystal display (LCD) shielding goggle without taking off according to an embodiment of the present invention.

As shown in FIG. 2, the welding mask according to this embodiment includes a welding face 100, a wearing band 200, an LCD shielding goggle 300, and a manipulation unit 400.

The welding face 100 is configured to cover the entirety of a user's face. Also, the welding face 10 has a predetermined transparent window 120 formed at the front thereof.

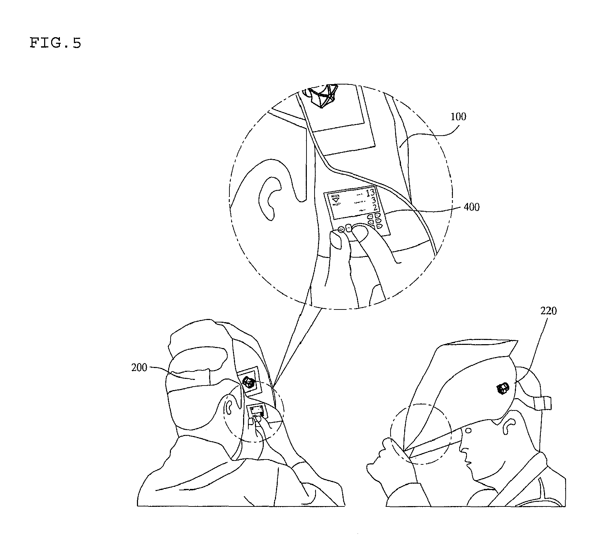

The wearing band 200 is configured to be worn on the user's hair. Also, the wearing band 200 is coupled to the welding face 100 via pivot shafts 220 (See FIG. 5) such that the welding face 100 is rotated upward/downward by a predetermined angle.

The LCD shielding goggle 300 includes an LCD panel 310 mounted at the rear of the transparent window 120 of the welding face 100 to protect the user's eyes from strong injurious light rays generated during welding.

The manipulation unit 400 is disposed spaced apart by a predetermined distance from the lower end of the LCD shielding goggle 300. Also, the manipulation unit 400 detects injurious light rays generated during welding and operates the LCD panel 310 accordingly.

FIG. 3 is a front view showing a welding mask according to another embodiment of the present invention.

In this embodiment, as shown in FIG. 3, the LCD panel 310 and the manipulation unit 400 may be connected to each other in a wire communication fashion.

Such a wire communication is electrical communication using a wire as an electrical transmission line. The wire communication has a disadvantage in that installation costs for the wire communication are very high, but has an advantage in that stable, high-quality electrical transmission is secured as compared with radio communication, and divergence from the electrical transmission line is possible.

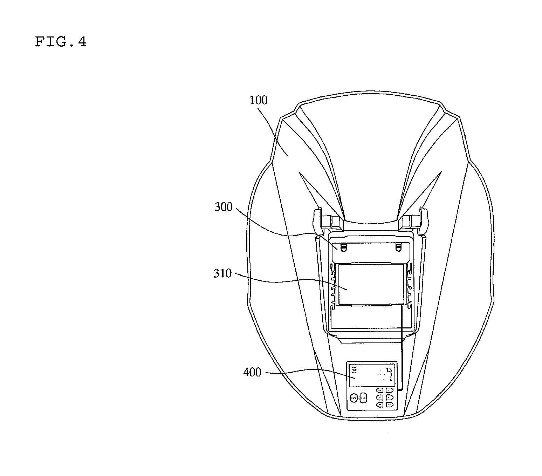

FIG. 4 is a front view showing a welding mask according to a further embodiment of the present invention.

In this embodiment, as shown in FIG. 4, the LCD panel 310 and the manipulation unit 400 may be connected to each other in a radio communication fashion.

Such a radio communication fashion is electrical communication using electromagnetic waves as a medium. To this end, the welding mask according to this embodiment may be provided with a communication unit including a radio transmitter, a radio receiver, a modem, and an amplifier, although not shown in the drawing.

In this case, the manipulation unit 400 may be configured in the form of a touch panel using an LCD or light emitting diodes (LED).

FIG. 5 is a view showing the use of the welding mask that can control the LCD shielding goggle without taking off according to the present invention.

As shown in FIG. 5, it is possible for a user to adjust sensitivity and shielding timing through the manipulation unit 400 without taking off the welding face 100. That is, it is possible for the user to perform adjustments and tests while wearing the welding face 100. Consequently, easy and convenient user manipulation is achieved.

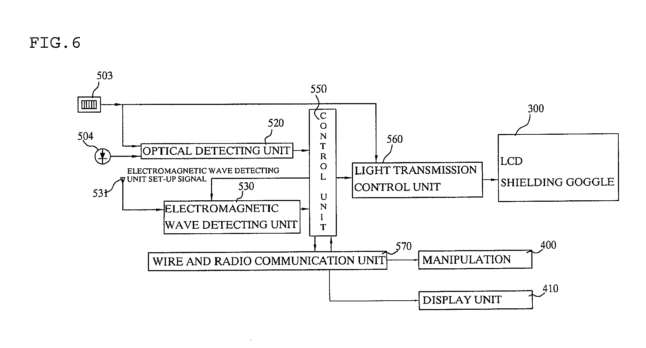

FIG. 6 is a block diagram showing a control circuit of the welding mask that can control the LCD shielding goggle without taking off according to the present invention.

As shown in FIG. 6, the welding mask includes a solar battery 503, a light sensing unit 504, an optical detection unit 520, an electromagnetic wave sensing unit 531, an electromagnetic wave detection unit 530, a control unit 550, a light transmission control unit 560, a display control unit 561, an LCD shielding goggle 300, a wire and radio communication unit 570, and a manipulation unit 400.

The optical detection unit 520 serves to detect light rays generated from a welding or cutting torch. The optical detection unit 520 compares a signal input from the light sensing unit 504 with the output of the solar battery 503 to detect the change of the light rays.

The electromagnetic wave detection unit 530 serves to detect electromagnetic waves generated from the welding or cutting torch. The electromagnetic wave detection unit 530 compares a signal, corresponding to the electromagnetic waves generated from the welding or cutting torch, input through the electromagnetic wave sensing unit 531, with a predetermined value set by resonance and filtering to detect a specific band of electromagnetic waves.

When the detection of light rays is commenced by the optical detection unit 520, the control unit 550 controls an electromagnetic wave detection unit drive signal to be applied to the electromagnetic wave detection unit 530 to monitor the change of an electromagnetic wave signal input from the output of the electromagnetic wave detection unit 530.

The light transmission control unit 560 controls the change of light transmissivity of the LCD shielding goggle 300 according to an output signal of the control unit 550.

A user inputs a command via the manipulation unit 400, which in turn inputs the user command to the control unit 550. At this time, a signal input to the manipulation unit 400 is converted into a wire signal or a radio signal by the wire and radio communication unit 570, and the converted signal is transmitted to the control unit 550.

The welding mask may further include a display unit 410 to display manipulation and operation states of equipment according to an output signal of the control unit 550. The display unit 410 may be provided together with the manipulation unit 400. Also, a signal from the display unit 410 may be converted into a wire signal or a radio signal, and the converted signal may be transmitted to the control unit 550.

As apparent from the above description, it is possible for the welding mask according to the present invention to enable a user to adjust shielding degree, sensitivity, open delay, grinding, cutting, etc. without taking off the welding face. That is, it is possible for the user to perform adjustments and tests while wearing the welding face. Consequently, the welding mask according to the present invention is easily adapted to the working environment of the user, thereby achieving easy and convenient user manipulation.

Although the exemplary embodiments of the present invention have been disclosed for illustrative purposes, those skilled in the art will appreciate that various modifications, additions and substitutions are possible, without departing from the scope and spirit of the invention as disclosed in the accompanying claims.

## Claims

1. A welding mask that can control a liquid crystal display (LCD) shielding goggle without taking off comprising:
a welding face configured to cover the entirety of a user's face, the welding face having a predetermined transparent window formed at the front thereof;
a wearing band configured to be worn on the user's hair, the wearing band being coupled to the welding face via pivot shafts such that the welding face is rotated upward/downward by a predetermined angle;
an LCD shielding goggle comprising an LCD panel mounted at the rear of the transparent window of the welding face to protect the user's eyes from strong injurious light rays generated during welding; and
a manipulation unit disposed spaced apart by a predetermined distance from the lower end of the LCD shielding goggle, the manipulation unit serving to detect injurious light rays generated during welding and operate the LCD panel accordingly.

2. The welding mask according to claim 1, wherein the LCD panel and the manipulation unit are connected to each other in a wire communication fashion.

3. The welding mask according to claim 1, wherein the LCD panel and the manipulation unit are connected to each other in a radio communication fashion.

4. The welding mask according to claim 1, wherein the manipulation unit is configured in the form of a touch panel using an LCD or light emitting diodes (LED).

5. The welding mask according to claim 1, further comprising a display unit to display manipulation and operation states of equipment.
